# EUROPÄISCHE PATENTSCHRIFT

(11) **EP 0 626 371 B1**
(45) Veröffentlichungstag und Bekanntmachung des Hinweises auf die Patenterteilung: **14.01.1998**
(21) Anmeldenummer: 94106195.4
(22) Anmeldetag: 21.04.1994
(51) Int. Cl.: C07C 409/26, C07C 409/24

(54) **Stabilisierte Percarbonsäurelösungen und Verfahren zu deren Herstellung**
Stabilized percarboxylic acid solutions and process for their preparation
Solutions d'acide percarboxylique stabilisées et procédé pour leur préparation

(30) Priorität: 26.05.1993 DE 4317420
(43) Veröffentlichungstag der Anmeldung: 30.11.1994
(73) Patentinhaber: Degussa Aktiengesellschaft, 60311 Frankfurt (DE)
(72) Erfinder: Täubl, Peter, A-9721 Weissenstein (AT)

(56) Entgegenhaltungen:
- WO-A-91/07375
- DD-A- 71 313
- DE-A- 2 451 904
- DE-B- 2 654 164
- US-A- 3 168 554
- CHEMICAL ABSTRACTS, vol. 67, no. 21, 1967, Columbus, Ohio, US; abstract no. 99587q, & SB. VED. PR., VYS. SK. CHEM.-TECHNOL., PARDUBICE, Bd.15, Nr.1, 1967 Seiten 113 - 122 S. HAVEL
- PATENT ABSTRACTS OF JAPAN vol. 12, no. 365 (C-532) (3212) 29. September 1988 & JP-A-63 119 457 (MITSUI TOATSU CHEM INC)
- PATENT ABSTRACTS OF JAPAN vol. 12, no. 365 (C-532) (3212) 29. September 1988 & JP-A-63 119 458 (MITSUI TOATSU CHEM INC)

## Beschreibung

Die Erfindung betrifft stabilisierte Percarbonsäurelösungen, enthaltend eine Mono- oder Dipercarbonsäure mit 1 bis 12 C-Atomen, insbesondere Peressigsäure, ein Lösungsmittel und eine synergistisch wirksame Stabilisatorkombination sowie ein Verfahren zu deren Herstellung, wobei die Stabilisatorkombination während der Herstellung der Percarbonsäurelösung anwesend ist oder ihr nach der Herstellung zugesetzt wird.

Percarbonsäurelösungen, insbesondere Peressigsäurelösung, finden vielseitige Anwendung. Wasserhaltige Percarbonsäurelösungen, wie etwa sogenannte Gleichgewichtsperessigsäure, finden beispielsweise Einsatz in Wasch-, Bleich- und Reinigungsmitteln sowie in mikrobiozid wirksamen Zusammensetzungen für Desinfektionszwecke. Wasserarme oder wasserfreie Percarbonsäurelösungen werden als Oxidationsmittel in der chemischen Synthese eingesetzt.

Percarbonsäurelösungen erfordern aufgrund ihrer chemischen Eigenschaften, insbesondere der in Gegenwart von Schwermetallen gegebenen Zersetzlichkeit, entsprechende Kenntnisse bei der Herstellung, Lagerung und Handhabung. Um Risiken zu vermeiden, enthalten derartige Lösungen in aller Regel ein oder mehrere Stabilisatoren. Aufgrund steigender Sicherheitsanforderungen besteht ein reges Interesse, wirksamere Stabilisatoren oder Stabilisatorkombinationen aufzufinden.

Ein aus der US-PS 2,609,391 bekannter Stabilisator für wäßrige Peressigsäurelösungen ist die Dipicolinsäure. Rohe Peressigsäurelösungen, wobei das Lösungsmittel ausgewählt ist aus der Reihe Essigsäure, Essigsäurealkylester, Aceton und bzw. oder Wasser oder deren Gemischen, lassen sich gemäß DE-PS 11 70 927 besser durch einen Zusatz von Picolinsäure stabilisieren.

Die Stabilisierung wäßriger Percarbonsäurelösungen mittels Pyrophosphat oder Pyrophosphorsäure lehrt die US-PS 2,347,434 und mittels eines polymeren Phosphats, wie etwa Natriumhexametaphosphat oder Natriumtripolyphosphat, die US-PS 2,590,856.

Um die Stabilität von Percarbonsäurelösungen zu erhöhen und/oder die Gesamtmenge an Stabilisatoren zu begrenzen, werden auch unterschiedliche Stabilisatorkombinationen verwendet. Beispielsweise lehrt die DE-AS 12 80 239 eine Kombination aus Chinolin und einem kondensierten Phosphat, die DE-OS 24 51 904 eine Kombination aus Dipicolinsäure und Natriumhexametaphosphat.

In der DE-OS 24 51 904 werden außer der in wäßriger Peressigsäurelösung und in rein organischer peressigsäurehaltiger Reaktionsmischung synergistisch wirksamen Kombination Dipicolinsäure/Natriumhexametaphosphat einige weitere Stabilisatorkombinationen offenbart, etwa die Kombination Dipicolinsäure/Pyrophosphorsäure mit je 200 ppm, bezogen auf die Lösung, in rein organischer Phase, welche sich ausweislich der angegebenen Stabilitätsdaten aber als nicht besonders wirksam erwies.

Gemäß DD-A 71 313 lassen sich Percarbonsäuren mit 0,01 bis 1 % 8-Hydroxychinolin sowie eine Kombination aus 0,001 bis 1 % 8-Hydroxychinolin und 0,001 bis 0,5 % Pyrophosphat oder Hexametaphosphat stabilisieren.

Eine weitere synergistisch wirksame Stabilisatorkombination für Peressigsäurelösungen, welche Peressigsäure, Essigsäure, Wasserstoffperoxid, Wasser und Mineralsäure enthalten, besteht gemäß der WO 91/07375 aus Dipicolinsäure und mindestens einer mit zweiwertigen Metallen zur Komplexbindung befähigten Phosphonsäure. Vorzugsweise enthalten derartige, durch Gleichgewichtseinstellung eines Gemischs aus Essigsäure, H₂O₂ und Wasser in Gegenwart einer Mineralsäure erhaltene Peressigsäurelösungen 0,1 bis 20 Gew.-% Phosphonsäure, wie z. B. 1-Hydroxyethan-1,1-diphosphonsäure, und 0,1 bis 5000 mg/l Dipicolinsäure. Auch gemäß der WO 91/13058 wird für Gleichgewichtsperessigsäure beispielgemäß eine gleichartige Kombination aus 1 % Dipicolinsäure und 1 % 1-Hydroxyethylidendiphosphat eingesetzt. Die Gesamtmenge an Stabilisatoren ist im Falle ausreichender Stabilisierung trotz der synergistischen Wirksamkeit der beiden Stabilisatorkomponenten sehr hoch und für manche Einsatzgebiete hinderlich.

Aufgabe der vorliegenden Erfindung ist die Bereitstellung stabilisierter Percarbonsäurelösungen, insbesondere wäßriger Percarbonsäurelösungen, mit einer synergistisch wirksamen Stabilisatorkombination. Die Einsatzmengen der Stabilisatoren sollten derart sein, daß mittels der Kombination die Menge an teurer Stabilisatorkomponente signifikant, d. h. auf weniger als ein Drittel der bisher benötigten Menge reduziert werden kann. Eine weitere Aufgabe besteht darin, stabilisierte wäßrige Peressigsäurelösungen bereitzustellen, welche den nach den UN-Richtlinien durchgeführten SADT-Text (self accelerated decomposition temperature) von "mindestens 60 °C" erfüllen.

Gelöst wird die Aufgabe durch stabilisierte Percarbonsäurelösungen, enthaltend eine Mono- oder Dipercarbonsäure mit 1 bis 12 C-Atomen, mindestens ein Lösungsmittel und mindestens je einen Stabilisator aus der Reihe (a) bestehend aus Pyrophosphorsäure, einem Alkalimetall- oder Ammoniumsalz derselben und (b) bestehend aus Dipicolinsäure oder einem Alkalimetall- oder Ammoniumsalz derselben, welche dadurch gekennzeichnet sind, daß sie 100 bis 500 ppm eines Stabilisators aus der Reihe (a) und 5 bis 80 ppm eines Stabilisators aus der Reihe (b), jeweils bezogen auf die Lösung, enthält.

Bei den Percarbonsäuren handelt es sich um wasserlösliche Percarbonsäuren mit 1 bis 4 C-Atomen, nämlich Perameisensäure, Peressigsäure, Perpropionsäure, Perbuttersäure und wenig oder wasserunlösliche Monopercarbonsäuren mit 5 bis 12 C-Atomen. Unter den Dipercarbonsäuren wird beispielhaft auf Diperoxybernsteinsäure und Dodecandiperoxydicarbonsäure hingewiesen.

Bevorzugte stabilisierte Percarbonsäurelösungen enthalten eine wasserlösliche Percarbonsäure, wie Peressigsäure oder Perpropionsäure. Besonders bevorzugt handelt es sich bei den stabilisierten Percarbonsäurelösungen um solche, welche außer einer wasserlöslichen Percarbonsäure, die ihr zugrundeliegende Carbonsäure, Wasserstoffperoxid und Wasser sowie gegebenenfalls eine Mineralsäure enthalten; zu dieser Klasse gehören im Handel erhältliche, sogenannte Gleichgewichtspercarbonsäuren mit einem Gehalt von insbesondere 1 bis 50 Gew.-% Percarbonsäure.

Besonders bevorzugte Gleichgewichtspercarbonsäurelösungen bestehen aus im wesentlichen
2,5 bis 43 Gew.-% Peressigsäure
5 bis 74 Gew.-% Essigsäure
1 bis 30 Gew.-% Wasserstoffperoxid
10 bis 60 Gew.-% Wasser
0,05 bis 1 Gew.-% Mineralsäure, insbesondere H₂SO₄
sowie der erfindungsgemäßen Stabilisatorkombination.

Unter den Pyrophosphorsäureverbindungen wird als Stabilisator Dinatriumdihydrogenpyrophosphat (DNPP) bevorzugt. Mit einer Einsatzmenge von 100 bis 500 ppm, bezogen auf die Percarbonsäurelösung, kann durch Zusatz bereits einer sehr niedrigen Menge Dipicolinsäure oder eines Salzes derselben, beispielsweise 5 bis 25 mg, eine wesentliche Stabilitätserhöhung erzielt werden.

Zu Stabilisatoren der Gruppe (b) gehören insbesondere Dipicolinsäure, Chinolinsäure, 2,4-Lutidinsäure, Dinicotinsäure sowie die Alkalimetall- und Ammoniumsalze der genannten Säuren. Besonders bevorzugt werden Dipicolinsäure sowie deren Salze. Auch Gemische aus mehreren Stoffen der Gruppe (b) können in den Percarbonsäurelösungen anwesend sein.

Bei Dipicolinsäure und ihre Salzen führt eine Einsatzmenge von 20 bis 80 ppm zu ausgezeichneten Stabilitätsdaten. Da es sich bei der Dipicolinsäure bzw. den Dipicolinaten um die gegenüber Pyrophosphorsäure und Pyrophosphaten viel teurere Stabilisatorkomponente handelt, führt die erfindungsgemäße Stabilisatorkombination zu einer wesentlichen Kosteneinsparung gegenüber alleiniger Verwendung einer solchen Menge Dipicolinsäure, wie sie zur Einreichung des für Gleichgewichtsperessigsäurelösung geforderten SADT-Tests "mindestens 60 °C" notwendig ist. Mittels alleiniger Verwendung von Pyrophosphorsäure oder Pyrophosphat kann der SADT-Test auch bei großer Stabilisatormenge bei Gleichgewichtsperessigsäure nicht erreicht werden, im Falle von Dipicolinsäure erfordert die Testerfüllung etwa 500 ppm.

Wie schon erwähnt, wurde die Kombination Dipicolinsäure/-Pyrophosphat in rein organischer Peressigsäurelösung in der DE-OS 24 51 904 genannt, jedoch gibt dieses Dokument weder eine Anregung, die dort für ein Epoxidations-Reaktiongemisch genannte Menge Dipicolinsäure wesentlich zu reduzieren, noch eine stofflich gleiche, aber bezüglich des Dipicolinsäureanteils wesentlich reduzierte Stabilisatorkombination zur Stabilisierung wäßriger Percarbonsäurelösungen zu verwenden.

Es ist bekannt, daß handelsübliche wäßrige Wasserstoffperoxidlösungen, wie sie für die Herstellung von Percarbonsäurelösungen verwendet werden, in stabilisierter Form in den Handel gelangen, wobei als Stabilisatoren auch Dipicolinsäure und Pyrophosphat gleichzeitig anwesend sein können. Die über das verwendete Wasserstoffperoxid in die Persäurelösung gelangende Menge Dipicolinsäure und Pyrophosphat liegt aber im Bereich von nur wenigen ppm und reicht zur erforderlichen Stabilisierung der Percarbonsäurelösung in gar keiner Weise aus.

Die erfindungsgemäßen Percarbonsäurelösungen können zusätzlich zur Stabilisatorkombination aus mindestens je einem Stoff der Gruppen (a) und (b) weitere übliche Stabilisatoren enthalten. Zu nennen sind beispielsweise Stabilisatoren aus der Reihe: Zinnverbindungen, wie Alkalistannate, Phosphonsäuren, wie z. B. Hydroxyethandiphosphonsäure und deren Salze, Chelatkomplexe bildende Hydroxy- und Aminocarbonsäuren sowie alkylierte Hydroxyaromaten.

Die erfindungsgemäßen Percarbonsäurelösungen lassen sich herstellen, indem mindestens je ein Stabilisator aus der Reihe (a) bestehend aus Pyrophosphorsäure, einem Alkalimetall- oder Ammoniumsalz derselben und (b) bestehend aus Dipicolinsäure oder einem Alkalimetall- oder Ammoniumsalz derselben der Percarbonsäure und mindestens ein Lösungsmittel enthaltenden Lösung oder vor der Lösungsherstellung einem Lösungsmittel, das ein wäßriges oder ein im wesentlichen wasserfreies organisches Lösungsmittel sein kann, oder Reaktionspartnern der Percarbonsäureherstellung zugegeben wird. Die Zugabemengen entsprechen denjenigen der erfindungsgemäßen stabilisierten Percarbonsäurelösungen.

Stabilisierte wäßrige Percarbonsäurelösungen lassen sich vorzugsweise dadurch herstellen, daß man eine Carbonsäure mit 1 bis 4 C-Atomen, insbesondere Essigsäure, mit wäßrigem Wasserstoffperoxid in Gegenwart eines stark sauren Katalysators, insbesondere Schwefelsäure umsetzt, wobei die Stabilisatoren einem der Einsatzstoffe oder dem Reaktionsgemisch vor, während und/oder nach der Umsetzung zugegeben werden.

Die erfindungsgemäßen Lösungen weisen eine überraschend gute Stabilität aus. Wäßrige Percarbonsäurelösungen, wie insbesondere Gleichgewichtsperessigsäurelösungen, erfüllen den geforderten SADT-Test, so daß derartige Lösungen die Transportvorschriften erfüllen. Die Herstellung der Lösungen ist einfach und lehnt sich an die an sich bekannten Verfahren an. Durch die Zusammensetzung der erfindungsgemäß erforderlichen Stabilisatorkombination gelangt man zu einer Kostenreduzierung.

Anhand der nachfolgenden Beispiele und Vergleichsbeispiele wird die Erfindung verdeutlicht.

### Beispiele B1 bis B4 und Vergleichsbeispiele VB1 bis VB6

Untersucht wurde die Zersetzungsrate verschiedener Qualitäten Gleichgewichtsperessigsäure (GPES), welche aus Peressigsäure, Essigsäure, Wasser, Wasserstoffperoxid, H₂SO₄ und Stabilisatoren bestanden, in Abwesenheit und in Gegenwart eines Zersetzungskatalysators (2 ppm V4A, zugegeben als Sulfat) durch 16stündiges Erhitzen der GPES auf 55 ^{o}C in einer Testvorrichtung aus Glas. Die Zersetzungsraten in Prozent pro Stunde, bezogen auf den Ausgangswert an Peressigsäure, folgen aus den Tabellen 1 und 2.

Eingesetzt wurden 40 gew.-%ige Gleichgewichtsperessigsäure mit einem H₂SO₄-Gehalt von 500 mg/kg (=GPES IA40), 40 gew.-%ige GPES mit einem H₂SO₄-Gehalt von 1 Gew.-% (=GPES SH40) und 10 gew.-%ige Gleichgewichtsperessigsäure mit einem H₂SO₄-Gehalt von 2500 mg/kg (=GPES 10), welche durch Verdünnen einer 40 gew.-%igen GPES mit 1 Gew.-% H₂SO₄ erhalten wurde. Die Stabilisatoren wurden in der in der Tabelle angegebenen Menge bereits während der Herstellung der GPES mit den Reaktionspartnern eingebracht. Zum Einsatz kamen Dipicolinsäure (DPS), Dinatriumdihydrogenpyrophosphat (DNPP) und Gemische DPS/DNPP.

**Tabelle 1**

| Stabilität von GPES: Gegenwart von 2 ppm V4A | | | |
|---|---|---|---|
| a) Stabilität von GPES IA40 | | | |
| Beispiel(B)Nr. und Vergleichsbeispiel(VB)Nr. | Stabilisator | Menge (ppm) | Zersetzungsrate (% / h) |
| VB 1 | ohne | - | 4,69 |
| VB 2.1 | DPS | 500 | 1,35 |
| VB 2.1 | DPS | 250 | 1,85 |
| VB 2.3 | DPS | 50 | 2,97 |
| VB 3.1 | DNPP | 500 | 2,05 |
| VB 3.2 | DNPP | 250 | 2,39 |
| B 1.1 | DPS/DNPP | 10/490 | 0,84 |
| B 1.2 | DPS/DNPP | 25/475 | 0,78 |
| B 1.3 | DPS/DNPP | 50/450 | 0,74 |
| B 2.1 | DPS/DNPP | 5/245 | 1,59 |
| B 2.2 | DPS/DNPP | 12/238 | 1,21 |
| B 2.3 | DPS/DNPP | 25/225 | 1,19 |
| B 2.3 | DPS/DNPP | 75/175 | 1,08 |

| b) Stabilität von GPES 10 | | | |
|---|---|---|---|
| VB 4 | DPS | 500 | 0,50 |
| B 3 | DPS/DNPP | 75/425 | 0,11 |

**Tabelle 2**

| Stabilität von GPES IA40 und GPES SH40 nach 14 tägiger Lagerung im Glasgefäß; Stabilitätstest 16 h bei 55 ^{o}C | | | | |
|---|---|---|---|---|
| Nr. | Stabilisator | Menge (ppm) | Zersetzungsrate (% / h) | |
| | | | IA 40 | SH 40 |
| VB 5 | ohne | - | 0,19 | 0,14 |
| VB 6 | DPS | 500 | 0,038 | 0,022 |
| B 4 | DPS/DNPP | 50/450 | 0,008 | 0,011 |

### Beispiel 5 Stabilität von 5 gew.-%iger GPES

Für die Herstellung dieser Lösung werden angesetzt: 3,14 kg vollentsalztes Wasser, 5,74 kg Wasserstoffperoxid 50 Gew.-%, 1,02 kg Essigsäure und 0,1 kg H₂SO₄ konz. Zu vom Reaktionsgemisch entnommenen 1 l-Proben wurden die in der Tabelle 3 angegebenen Stabilisatoren zugefügt. Nach jeweils 2-tägigem Stehenlassen des stabilisierten Reaktionsgemischs wurde die Stabilität in einer üblichen Vorrichtung zur gasvolumetrischen Bestimmung des bei der Zersetzung bei 55 ^{o}C freigesetzten Sauerstoffs geprüft, wobei den Proben zunächst 1 mg Eisen in Form von Eisensulfat pro 1 Lösung zugefügt wurde. Tabelle 3 enthält die Ergebnisse: Hiernach wirkt das erfindungsgemäße Stabilisatorsystem synergistisch.

**Tabelle 3**

| Nr. | Stabilisator | Menge (mg/l) | Zersetzungsrate (ml O₂ / h) |
|---|---|---|---|
| VB 7 | DPS | 50 | 540 |
| VB 8 | DNPP | 450 | 0,9 |
| B 5 | DPS/DNPP | 50/450 | 0,5 |

Bei der adiabatischen Warmlagerung besteht die Probe gemäß Beispiel 5 den SADT-Test, so daß die Angabe "mindestens 60 ^{o}C" erlaubt ist (UN-Richtlinien zur UN-Klasse 5.2; siehe Orange Book / Transparent of Dangerous Goods; Tests and Criteria, Part II, Section 4, 1990, pages 205-209). VB 7 und VB 8 bestehen den Test nicht.

## Patentansprüche

1. Stabilisierte Percarbonsäurelösunqen, enthaltend eine Mono- oder Dipercarbonsäure mit 1 bis 12 C-Atomen, mindestens ein Lösungsmittel und mindestens je einen Stabilisator aus der Reihe (a) bestehend aus Pyrophosphorsäure, einem Alkalimetall- oder Ammoniumsalz derselben und (b) bestehend aus Dipicolinsäure oder einem Alkalimetall- oder Ammoniumsalz derselben,
dadurch gekennzeichnet,
daß sie 100 bis 500 ppm eines Stabilisators aus der Reihe (a) und 5 bis 80 ppm eines Stabilisators aus der Reihe (b), jeweils bezogen auf die Lösung, enthält.

2. Stabilisierte Percarbonsäurelösung nach Anspruch 1,
dadurch gekennzeichnet,
daß sie eine Percarbonsäure mit 1 bis 4 C-Atomen, insbesondere Peressigsäure, die ihr zugrundeliegende Carbonsäure, Wasserstoffperoxid, Wasser, mindestens zwei Stabilisatoren und gegebenenfalls eine Mineralsäure enthält.

3. Stabilisierte Percarbonsäurelösung nach Anspruch 2,
dadurch gekennzeichnet,
daß es sich um eine Gleichgewichtsperessigsäure handelt, vorzugsweise eine solche mit einem Gehalt von
2,5 bis 43 Gew.-% Peressigsäure
5 bis 74 Gew.-% Essigsäure
1 bis 30 Gew.-% Wasserstoffperoxid
10 bis 60 Gew.-% Wasser
0,05 bis 1 Gew.-% Mineralsäure, insbesondere H₂SO₄.

4. Stabilisierte Percarbonsäurelösung nach einem der Ansprüche 1 bis 3,
dadurch gekennzeichnet,
daß sie als Stabilisator der Gruppe (a) Dinatriumdihydrogenpyrophosphat (DNPP) enthält.

5. Verfahren zur Herstellung einer stabilisierten Percarbonsäurelösung, enthaltend eine Mono- oder Dipercarbonsäure mit 1 bis 12 C-Atomen und mindestens ein Lösungsmittel, durch Zugabe von mindestens je einem Stabilisator aus der Reihe (a) bestehend aus Pyrophosphorsäure, einem Alkalimetall- oder Ammoniumsalz derselben und (b) bestehend aus Dipicolinsäure oder einem Alkalimetall- oder Ammoniumsalz derselben zur genannten Lösung oder zum Lösungsmittel oder den Reaktionspartnern der Percarbonsäure vor der Herstellung derselben,
dadurch gekennzeichnet,
daß man einen Stabilisator aus der Reihe (a) in einer Menge von 100 bis 500 ppm und einen Stabilisator aus der Reihe (b) in einer Menge von 5 bis 80 ppm, jeweils bezogen auf die Lösung, zugibt.

6. Verfahren zur Herstellung einer stabilisierten Percarbonsäurelösung gemäß Anspruch 2 nach Anspruch 5,
dadurch gekennzeichnet,
daß man eine Carbonsäure mit 1 bis 4 C-Atomen, insbesondere Essigsäure, mit wäßrigem Wasserstoffperoxid in Gegenwart eines stark sauren Katalysators, insbesondere Schwefelsäure umsetzt, wobei die Stabilisatoren einem der Einsatzstoffe oder dem Reaktionsgemisch vor, während und/oder nach der Umsetzung zugegeben werden.

## Claims

1. Stabilised percarboxylic acid solutions containing a mono- or dipercarboxylic acid having 1 to 12 C atoms, at least one solvent and at least one each of the stabilisers from the range (a) consisting of pyrophosphoric acid, an alkali metal salt thereof or ammonium salt thereof and (b) consisting of dipicolinic acid or an alkali metal salt thereof or ammonium salt thereof,
characterised in that
they contain 100 to 500 ppm of a stabiliser from the series (a) and 5 to 80 ppm of a stabiliser from the series (b), in each case relative to the solution.

2. Stabilised percarboxylic acid solution according to claim 1,
characterised in that
it contains a percarboxylic acid having 1 to 4 C atoms, in particular peracetic acid, the underlying carboxylic acid, hydrogen peroxide, water, at least-two stabilisers and optionally a mineral acid.

3. Stabilised percarboxylic acid solution according to claim 2,
characterised in that
it is a peracetic acid at equilibrium, preferably such an acid having a content of
2.5 to 43 wt.% of peracetic acid
5 to 74 wt.% of acetic acid
1 to 30 wt.% of hydrogen peroxide
10 to 60 wt.% of water
0.05 to 1 wt.% of mineral acid, in particular H₂SO₄.

4. Stabilised percarboxylic acid solution according to one of claims 1 to 3,
characterised in that
it contains disodium dihydrogen pyrophosphate (DNPP) as the stabiliser from group (a).

5. Process for the production of a stabilised percarboxylic acid solution containing a mono- or dipercarboxylic acid having 1 to 12 C atoms and at least one solvent by adding at least one each of the stabilisers from the range (a) consisting of pyrophosphoric acid, an alkali metal salt thereof or ammonium salt thereof and (b) consisting of dipicolinic acid or an alkali metal salt thereof or ammonium salt thereof to the stated solution or to the solvent or to the reactants of the percarboxylic acid before the production thereof,
characterised in that
a stabiliser from the series (a) is added in a quantity of 100 to 500 ppm and a stabiliser from the series (b) is added in a quantity of 5 to 80 ppm, in each case relative to the solution.

6. Process in accordance with claim 5 for the production of a stabilised percarboxylic acid solution according to claim 2,
characterised in that
a carboxylic acid having 1 to 4 C atoms, in particular acetic acid, is reacted with aqueous hydrogen peroxide in the presence of a strongly acidic catalyst, in particular sulphuric acid, wherein the stabilisers are added to one of the starting materials or to the reaction mixture before, during and/or after the reaction.

## Revendications

1. Solutions d'acide percarboxylique stabilisées contenant un acide mono- ou dipercarboxylique comportant 1 à 12 atomes de carbone, au moins un solvant et au moins un stabilisant respectivement de la série (a), comprenant l'acide pyrophosphorique, un sel alcalin ou ammoniaque de celui-ci, et (b), comprenant l'acide dipicolinique ou un sel alcalin ou ammoniaque de celui-ci,
caractérisées en ce qu'
elles contiennent 100 jusqu'à 500 ppm d'un stabilisant de la série (a) et 5 jusqu'à 80 ppm d'un stabilisant de la série (b), respectivement par rapport à la solution.

2. Solution d'acide percarboxylique stabilisée selon la revendication 1,
caractérisée en ce qu'
elle contient un acide percarboxylique comportant 1 à 4 atomes de carbone, en particulier l'acide peracétique, l'acide qui en est la base, du peroxyde d'hydrogène, de l'eau, au moins deux stabilisants et le cas échéant un acide minéral.

3. Solution d'acide percarboxylique stabilisée selon la revendication 2,
caractérisée en ce qu'
il s'agit d'un acide peracétique à équilibre, laquelle présente de préférence une teneur de
2,5 jusqu'à 43 % en poids d'acide peracétique
5 jusqu'à 74 % en poids d'acide acétique
1 jusqu'à 30 % en poids de peroxyde d'hydrogène
10 jusqu'à 60 % en poids d'eau
0,05 jusqu'à 1 % en poids d'acide minéral, en particulier le H₂SO₄.

4. Solution d'acide percarboxylique stabilisée selon une des revendications 1 à 3,
caractérisée en ce qu'
elle contient en tant que stabilisant du groupe (a) le dihydrogénopyrophosphate de sodium (DNPP).

5. Procédé pour la fabrication d'une solution d'acide percarboxylique stabilisée contenant un acide mono- ou dipercarboxylique comportant 1 à 12 atomes de carbone et au moins un solvant, au moyen d'une addition d'au moins un stabilisant respectivement de la série (a) comprenant l'acide pyrophosphorique, un sel alcalin ou ammoniaque de celui-ci et (b) comprenant l'acide dipicolinique ou un sel alcalin ou ammoniaque de celui-ci, à la solution mentionnée ou au solvant ou aux réactifs de la fabrication de l'acide percarboxylique avant la fabrication de cette dernière,
caractérisé en ce que
l'on ajoute un stabilisant de la série (a) en une quantité de 100 jusqu'à 500 ppm et un stabilisant de la série (b) en une quantité de 5 jusqu'à 80 ppm, respectivement par rapport à la solution.

6. Procédé de fabrication d'une solution d'acide percarboxylique stabilisée conformément à la revendication 2 selon la revendication 5,
caractérisé en ce que
l'on transforme un acide carboxylique comportant 1 à 4 atomes de carbone, en particulier l'acide acétique, avec du peroxyde d'hydrogène aqueux en présence d'un catalyseur fortement acide, en particulier l'acide sulfurique, où les stabilisants sont ajoutés à un des réactifs ou au mélange réactionnel avant, pendant ou après la transformation.
